# EUROPÄISCHE PATENTSCHRIFT

(11) **EP 0 585 555 B1**
(45) Veröffentlichungstag und Bekanntmachung des Hinweises auf die Patenterteilung: **06.10.1999**
(21) Anmeldenummer: 93110581.1
(22) Anmeldetag: 02.07.1993
(51) Int. Cl.: G01N 3/04, G01N 3/00, G01N 33/36

(54) **Mechanismus zum Einlegen eines Fadens in ein Garnprüfgerät**
Mechanism for inserting a yarn in a yarn tester
Mécanisme pour insérer un fil dans un appareil de contrôle de fil

(30) Priorität: 10.07.1992 CH 218792
(43) Veröffentlichungstag der Anmeldung: 09.03.1994
(73) Patentinhaber: ZELLWEGER LUWA AG, 8610 Uster (CH)
(72) Erfinder: Etter, Heinz, CH-8400 Winterthur (CH); Schlaepfer, Willi, CH-8320 Fehraltorf (CH)
(74) Vertreter: Ellenberger, Maurice

(56) Entgegenhaltungen:
- EP-A- 0 403 988
- AT-B- 181 101
- DE-A- 1 905 161
- DE-A- 3 012 499

## Beschreibung

Die vorliegende Erfindung betrifft einen Mechanismus zum Einlegen eines Fadens in ein Garnprüfgerät, welches eine Prüfstrecke und ein Uebernahmeorgan für den eingelegten Faden aufweist, mit einem Einlegearm und einer Klemme zur Vorlage des Fadens an diese.

Ein Garnprüfgerät dieser Art ist beispielsweise die in der EP-A-403 988 beschriebene Reissfestigkeitprüfanlage USTER TENSOJET (USTER - eingetragenes Warenzeichen der Zellweger Uster AG), bei der die Dehnung des Prüfguts durch zwei voneinander beabstandete, rotierende Walzenpaare erfolgt, zwischen deren Walzen ein periodisch sich öffnender und schliessender Klemmspalt für das Prüfgut gebildet ist. Diese Prüfanlage zeichnet sich unter anderem durch eine sehr hohe Prüfgeschwindigkeit aus, die, insbesondere bei der Prüfung von Garn verschiedener Spulen, einen entsprechenden Einlegemechanismus für die zu prüfenden Fäden erfordert.

In der zitierten EP-A-403 988 ist als geeigneter Einlegemechanismus der vom USTER TENSORAPID her bekannte Mechanismus erwähnt und dargestellt, der einen Einlegearm für den Faden enthält. Dieser Einlegearm, der im Prinzip in der US-A-4 319 493 beschrieben ist, ist in einer Kulisse gelagert, die in einer vertikal, in Fadenlaufrichtung, orientierten Schiene geführt ist, so dass der Arm mit seinem Kopf bei jedem Einlegezyklus eine Bahn längs einer geschlossenen, annähernd rechteckigen Kurve beschreibt. Der Arm trägt an seinem Kopf eine gesteuerte Fadenklemme, erfasst mit dieser den vorgelegten Faden, legt ihn in die Prüfanlage ein und gibt ihn nach der Uebernahme durch diese frei.

Praktische Versuche haben gezeigt, dass dieser Einlegemechanismus relativ langsam arbeitet und dadurch die Prüfgeschwindigkeit limitiert. Dies ist dadurch bedingt, dass der Arm beim Einlegen des Fadens eine relativ komplizierte Bahn beschreibt, und dass bei jedem Einlegezyklus die am Arm angeordnete Fadenklemme zweimal aktiviert werden muss. Ausserdem ist auch die Uebergabe des durch den Einlegearm an seinem Kopfteil ausgezogenen Fadens an das Uebernahmeorgan relativ kompliziert und erfordert eine genaue Positionierung und Synchronisation zwischen Uebernahmeorgan und Fadenklemme des Einlegearms. Dieser bekannte Einlegemechanismus beansprucht auch relativ viel Platz, was nicht nur ein klobiges Aussehen, sondern auch eine Steigerung der Kosten bewirkt.

Durch die Erfindung soll nun ein Einlegemechanismus angegeben werden, der eine hohe Einlegefrequenz und eine möglichst einfache Fadenübergabe an das Uebernahmeorgan ermöglicht, und der sich durch einen geringen Platzbedarf auszeichnet.

Diese Aufgabe wird erfindungsgemäss dadurch gelöst, dass der Einlegearm als ortsfest gelagerter Schwenkarm ausgebildet und der Faden bis zur Uebergabe an das Uebernahmeorgan in der Klemme fixiert und dem Uebernahmeorgan als Schlaufe zugeführt ist.

Die ortsfeste Lagerung des Einlegearms und dessen Ausbildung als Schwenkarm bedeutet, dass der dem Einlegearm vorgelegte Faden in einer einfachen Schwenkbewegung ausgezogen und dem Uebernahmeorgan zugeführt wird. Der Schlaufenauszug bedeutet, dass keine Fadenklemme am Einlegearm selbst erforderlich ist, so dass dieser sehr einfach ausgebildet werden kann. Die Uebergabe des schlaufenförmigen Fadens an das Uebernahmeorgan erfordert ebenfalls nur einen geringen Aufwand. Wenn beispielsweise das Uebernahmeorgan als Luftdüse, vorzugsweise als Saugdüse, ausgebildet ist, dann genügt es, den Faden quer zu deren Eintrittsöffnung zu positionieren, was mit einer Fadenschlaufe sehr einfach ist. Der geringe Platzbedarf des erfindungsgemässen Einlegemechanismus ist offensichtlich.

Im folgenden wird die Erfindung anhand eines Ausführungsbeispiels und der Zeichnungen näher erläutert; es zeigt:
- Fig. 1: eine ausschnittweise Vorderansicht des einen erfindungsgemässen Einlegemechanismus enthaltenden Teils einer Reissfestigkeitsprüfanlage; und
- Fig. 2: einen Schnitt nach der Linie II-II von Fig. 1.

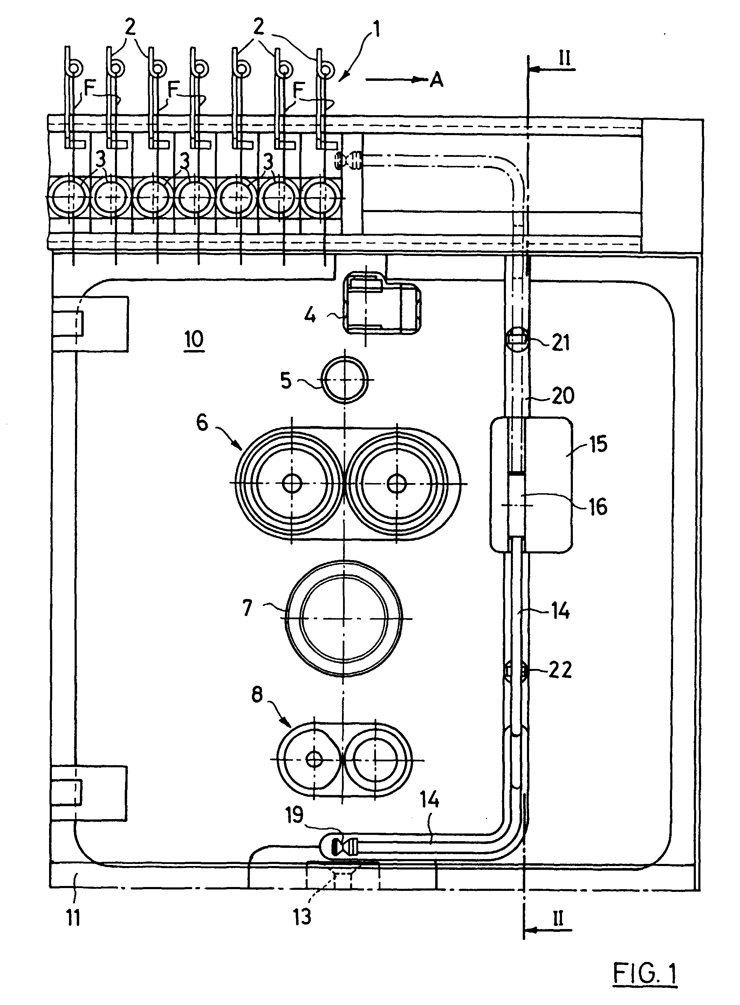

Fig. 1 zeigt eine Vorderansicht des Zuführteils einer Reissfestigkeitsprüfanlage, wie sie in der EP-A-403 988 beschrieben ist. Der zu prüfende Faden F wird dem Zuführteil von oben zugeführt, durchlauft diesen von oben nach unten und gelangt anschliessend in die nicht dargestellte Einlaufpartie des eigentlichen Prüfteils der Prüfanlage. Bezüglich des Prüfteils wird auf die EP-A-403 988 verwiesen.

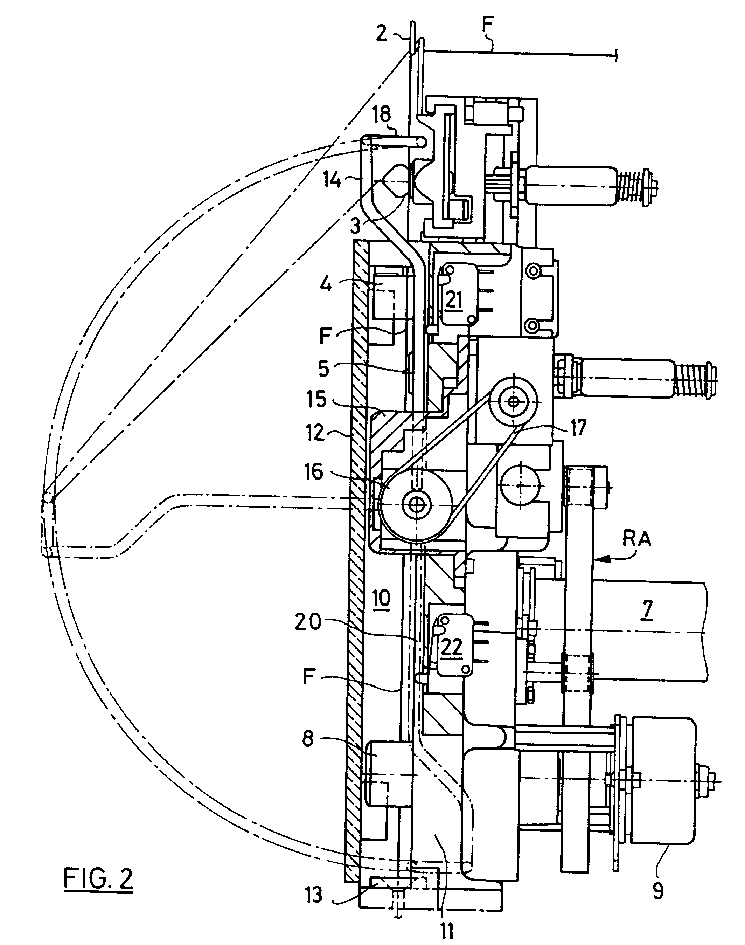

Darstellungsgemäss ist an der oberen Kante des Zuführteils ein Garnwechsler 1 angeordnet, der aus nebeneinander angeordneten Fadenführungsösen 2 und Garnklemmen 3 besteht. Diese bilden eine in Richtung des Pfeiles A schrittweise verschiebbare Klemmleiste mit beispielsweise je 12 Fadenführungsösen 2 und Garnklemmen 3. Auf einem Spulenspeicher der beispielsweise in der CH-Patentanmeldung Nr. 2072/90 beschriebenen Art werden die zu prüfenden Spulen aufgesteckt und die Fäden werden von Hand in die Führungsösen 2 und Garnklemmen 3 eingelegt und sind zwischen diesen gespannt. Wie insbesondere Fig. 2 entnommen werden kann, können die eingespannten Fäden an ihrem freien Stück zwischen Fadenführungsöse 2 und Garnklemme 3 von einem geeigneten Einlegeorgan ergriffen und durch die Fadenführungsösen 2 abgezogen werden.

Nach dem Garnwechsler 1 ist in Fadenlaufrichtung eine Schere 4 angeordnet, nach dieser ein Changierorgan 5 und anschliessend an dieses ein Paar motorisch antreibbarer Lieferwalzen 6, mit denen der Faden F im Prüfbetrieb kontinuierlich von seinem Vorrat abgezogen wird. Das Changierorgan 5 ist senkrecht zur Zeichenebene hin- und hergehend angetrieben und führt dabei den Faden F zwischen den Lieferwalzen 6 vorwärts und rückwärts, was eine gleichmässige Beanspruchung der Lieferwalzen 6 und damit einen einwandfreien Fadenabzug gewährleistet.

Da die im Prüfteil der Reissfestigkeitsprüfanlage erfolgenden Schritte Klemmen, Dehnen, Reissen und Freigabe des Fadens diskontinuierlich ablaufen, der Faden F aber durch die Lieferwalzen 6 kontinuierlich abgezogen wird, ist zwischen den Lieferwalzen 6 und dem Prüfteil ein Fadenspeicher vorgesehen. Dieser umfasst einen pneumatischen Fadenspeicher 7, wie er beispielsweise von Luftdüsenwebmaschinen her bekannt ist, und eine gesteuerte Fadenklemme 8, die aus einer kontinuierlich angetriebenen Steuerwalze mit abgestuftem Umfang und aus einer frei drehbaren Gegenwalze besteht. Die gesteuerte Fadenklemme 8 ermöglicht eine gesteuerte Leerung des Fadenspeichers 7, wobei wegen des abgestuften Umfangs der Steuerwalze der Faden F durch die Fadenklemme 8 entweder geklemmt oder freigegeben ist.

Der Antrieb der Steuerwalze ist über einen Riemenantrieb RA mit demjenigen der Lieferwalzen 6 gekoppelt. Mit dem Bezugszeichen 9 ist ein Inkrementalgeber für die Steuerwalze der Fadenklemme 8 bezeichnet. Wie Fig. 2 weiter zu entnehmen ist, sind die Organe Schere 4, Changierorgan 5, Lieferwalzen 6, Fadenspeicher 7 und gesteuerte Fadenklemme 8 in einem flachen Gehäuse angeordnet, das durch eine ausgefräste Abstufung 10 eine Platte 11 gebildet und nach vorne durch eine transparente Schwenktüre 12 abgeschlossen ist. Der Fadenspeicher 7 erstreckt sich vom Boden dieser Abstufung nach hinten und die anderen genannten Organe übertragen den Boden und reichen bis knapp an die Schwenktüre 12. In die in den Fig. 1 und 2 untere Seitenwand der Abstufung 10 ist eine Saugdüse 13 eingesetzt deren Längsachse mit dem Faden F fluchtet, und die zur Förderung des Fadens F aus dem Fadenspeicher 7 in einen Fadenführungskanal des Prüfteils der Prüfanlage dient.

Zu Beginn einer Prüfung wird der betreffende Faden F in den Zuführteil eingelegt und dabei bis an die Düse 13 gebracht, von der er dann pneumatisch in den Prüfteil gefördert wird. Das Einlegen erfolgt durch einen Mechanismus, der im wesentlichen aus zwei Komponenten besteht, aus dem schon beschriebenen Garnwechsler 1 und aus einem Einlegearm 14. Der letztere ist nadelförmig ausgebildet und hat eine annähernd L-förmige Gestalt mit einem längeren und einem von diesem abgesetzten kürzeren Schenkel. Der längere Schenkel ist auf einem in einem Lagerbock 15 gelagerten scheiben- oder klotzartigen Träger 16 befestigt, der über einen Zahnriemen 17 (Fig. 2) motorisch antreibbar ist. Der kürzere Schenkel trägt an seinem freien Ende einen Finger 18, an dem ein Fadenführer 19 befestigt ist. Der längere Schenkel erstreckt sich in Fadenlaufrichtung, der kürzere Schenkel quer zu dieser.

Die Fig. 1 und 2 zeigen den Einlegearm 14 in jeweils zwei Stellungen, und zwar in der Ruhestellung und in der Arbeitsstellung, die einen Winkelabstand von 180° aufweisen, und zwischen denen der Einlegearm 14 durch seinen Antrieb verschwenkbar ist. In Fig. 1 ist die Ruhestellung und in Fig. 2 die Arbeitsstellung mit vollen Linien eingezeichnet. In beiden Stellungen liegt der Fadenführer 19 etwa im Niveau des Bodens der Abstufung 10 und somit hinter dem Faden F. In der Arbeitsstellung ragt der Einlegearm 14 oben aus der Abstufung 10 heraus und der Fadenführer 19 ist im Garnwechsler 1 zwischen der Fadenführungsöse 2 und der Garnklemme 3 positioniert. In der Ruhestellung liegt der Fadenführer 19 knapp oberhalb der Saugdüse 13. Eine in den Boden der Abstufung 10 eingefräste, den Einlegearm 14 umgebende Nut 20 mit abgestufter Tiefe ermöglicht das Verschwenken des Einlegearms 14 bis in das Niveau des genannten Bodens. Im Bereich des an den Träger 16 anschliessenden Teils des längeren Schenkels des Einlegearms 14 ist im Boden der Nut 20 zu beiden Seiten des Trägers 16 je ein diesen Boden überragender Mikroschalter 21, 22 angeordnet, durch dessen Betätigung der über den Zahnriemen 17 den Träger 16 antreibende Motor gestoppt und ein das Erreichen der Arbeits- bzw. Ruhestellung des Einlegearms 14 anzeigendes Signal erzeugt wird.

Bei dem in Fig. 1 dargestellten Funktionszustand ist die Anlage zur Prüfung einer Reihe von Fäden F vorbereitet; die Fäden wurden von Hand in den Garnwechsler 1 eingelegt und der Einlegearm 14 befindet sich in seiner Ruhestellung, in der er seit der letzten Einlegeoperation verharrt. Die Lieferwalzen 6 sind etwas auseinandergeschwenkt, so dass zwischen ihnen ein Spalt gebildet ist, und die gesteuerte Fadenklemme 8 ist offen. Mit dem Drücken des Startknopfes wird einerseits die Saugdüse 13 aktiviert, und andererseits schwenkt der Einlegearm 14 in seine Arbeitsposition (Fig. 2), deren Erreichen durch den Mikroschalter 21 signalisiert wird. Nun wird der Garnwechsler 1 um einen Schritt in Richtung des Pfeiles A nach rechts verschoben, wodurch der Faden F der vordersten, aus Fadenführungsöse 2 und Garnklemme 3 gebildeten, Zelle des Garnwechslers 1 dem Fadenführer 19 vorgelegt wird. Die Länge des Arbeitsschritts des Garnwechslers 1 entspricht dem halben gegenseitigen Abstand der in diesem eingespannten Fäden. Ist die Vorlegeposition erreicht, was anhand der Verschiebung des Garnwechslers 1 detektiert wird, wird der Antriebsmotor des Trägers 16 eingeschaltet, wodurch der Einlegearm 14 aus seiner Arbeits- in die Ruhestellung geschwenkt wird. Dabei beschreibt der Fadenführer 19 den in Fig. 2 strichpunktiert eingezeichneten Halbkreis und erfasst unmittelbar nach Verlassen der Arbeitsstellung den ihm vorgelegten Faden F. Da dieser Faden in der Garnklemme 3 fixiert ist, wird er bei der Schwenkbewegung des Einlegearms 14 als Schlaufe aus der Fadenführungsöse 2 herausgezogen, wobei sein zwischen Garnklemme 3 und dem Fadenführer 19 gespanntes Teilstück wie ein Zeiger gegen die Verbindungsachse zwischen dem Spalt zwischen den Lieferwalzen 6 und dem offenen Klemmspalt der gesteuerten Fadenklemme 8 bewegt wird.

Nach Erreichen von etwa 170° des 180° Schwenkwinkels wird das genannte Teilstück des Fadens F zuerst in die gesteuerte Fadenklemme 8 und dann zwischen die Lieferwalzen 6 eingelegt bis schliesslich der Fadenführer 19 die Eintrittsöffnung der Saugdüse 13 überstreicht und der Faden F dem Sog der Düse ausgesetzt wird. Unmittelbar darauf erreicht der Einlegearm 14 seine Ruhestellung, was durch den Mikroschalter 22 signalisiert wird. Dadurch wird einerseits die Garnklemme 3 geöffnet und andererseits werden die Lieferwalzen 6 gestartet und gegeneinander geschwenkt und der Faden F wird nun durch die letzteren von seinem Vorrat abgezogen und durch die Saugdüse 13 der Prüfstrecke zugeführt. Gleichzeitig wird der Fadenspeicher 7 aktiviert.

Nach Abschluss der Prüfung, deren Dauer beispielsweise an einem Teil der Prüfanlage bildenden Bedienungsgerät eingestellt oder von diesem automatisch gesteuert wird, wird die Garnklemme 3 geschlossen und der Garnwechsler 1 um einen Arbeitsschritt in Richtung des Pfeiles A verschoben. Gleichzeitig werden die Lieferwalzen 6 voneinander weggeschwenkt und angehalten. Ebenso wird der Antrieb der gesteuerten Fadenklemme 8 gestoppt, und zwar so, dass die Klemme geöffnet ist, und der Fadenspeicher 7 wird ebenfalls abgeschaltet. Der Faden F liegt nun im Wirkungsbereich der Schere 4 und wird von dieser abgeschnitten. Das abgeschnittene Fadenende wird von der Düse 13 abgesaugt. Damit ist die Anlage für eine weitere Prüfung bereit, die mit dem Zurückschwenken des Einlegearms 14 in die Arbeitsstellung beginnt.

Selbstverständlich wird jeweils vor dem Zurückschwenken des Einlegearms 14 in die Arbeitsstellung die Schwenktüre 12 geöffnet; diese bleibt solange offen, bis der Einlegearm 14 wieder seine Ruhestellung erreicht hat und wird dann automatisch geschlossen. Das Oeffnen der Tür 12 wird durch die Betätigung des Startknopfs ausgelöst, das Schliessen durch den Mikroschalter 22.

## Patentansprüche

1. Mechanismus zum Einlegen eines Fadens in ein Garnprüfgerät, welches eine Prüfstrecke und ein Uebernahmeorgan (13) für den eingelegten Faden (F) aufweist, mit einem Einlegearm (14) und einer Klemme (3) zur Vorlage des Fadens (F) an diese, dadurch gekennzeichnet, dass der Einlegearm (14) als ortsfest gelagerter Schwenkarm ausgebildet und der Faden (F) bis zur Uebergabe an das Uebernahmeorgan (13) in der Klemme (3) fixiert und dem Uebernahmeorgan als Schlaufe zugeführt ist.

2. Mechanismus nach Anspruch 1, dadurch gekennzeichnet, dass in Fadenlaufrichtung vor der Klemme (3) eine Fadenführung (2) angeordnet und der Faden (F) zwischen dieser und der Klemme gespannt und in diesem Bereich dem Einlegearm (14) vorgelegt ist.

3. Mechanismus nach Anspruch 2, dadurch gekennzeichnet, dass Klemme (3) und Fadenführung (2) von einem gemeinsamen Träger getragen sind, und dass eine Mehrzahl von derartigen Klemmen, Fadenführungen und Trägern vorgesehen ist, welche eine verschiebbare Klemmleiste (1) bilden.

4. Mechanismus nach einem der Ansprüche 1 bis 3, dadurch gekennzeichnet, dass das Uebernahmeorgan (13) durch eine Luftdüse, vorzugsweise eine Saugdüse, gebildet und das der Faden (F) durch den Einlegearm (14) in den Bereich der Eintrittsöffnung der Düse geführt ist.

5. Mechanismus nach Anspruch 4, dadurch gekennzeichnet, dass der Einlegearm (14) abgewinkelt, vorzugsweise L-förmig ausgebildet ist und zwei Schenkel aufweist und an seinem einen Ende an einem antreibbaren Träger (16) befestigt und an seinem anderen Ende mit einem Fadenführer (19) versehen ist.

6. Mechanismus nach Anspruch 5, dadurch gekennzeichnet, dass der mit dem Träger (16) verbundene Schenkel des Einlegearms (14) parallel zur Verbindungsachse zwischen der Klemme (3) und der Düse (13) und der andere Schenkel rechtwinklig dazu orientiert ist, und dass der Weg des Einlegearms bei seiner Schwenkbewegung von der Klemme zur Düse etwa 180° beträgt.

7. Mechanismus nach Anspruch 6, dadurch gekennzeichnet, dass durch den Einlegearm (14) betätigbare Schalter (21, 22) zur Detektion des Erreichens der jeweiligen Endposition der Schwenkbewegung vorgesehen sind.

8. Mechanismus nach den Ansprüchen 3 und 7, dadurch gekennzeichnet, dass der Träger (16) des Einlegearms (14), die Klemmleiste (1) und die Düse (13) auf einer gemeinsamen Platte (11) angeordnet sind.

9. Mechanismus nach Anspruch 8, dadurch gekennzeichnet, dass die Platte (11) mit einer Vertiefung (10) versehen ist, welche eine Nut (20) zur Aufnahme des Einlegearms (14) aufweist, und dass die Vertiefung nach oben durch eine schwenkbare Klappe oder Türe (12) abgeschlossen ist.

10. Mechanismus nach Anspruch 9, dadurch gekennzeichnet, dass der Einlegearm (14) nach dem Einlegevorgang in seiner Ruhestellung mit dem Fadenführer (19) im Bereich der Düse (13) positioniert ist.

11. Mechanismus nach Anspruch 10, dadurch gekennzeichnet, dass bei Inbetriebnahme des Mechanismus eine Verschwenkung des Einlegearms (14) aus der Ruhestellung in die Arbeitsstellung mit dem Fadenführer (19) im Bereich der Klemmleiste (1) erfolgt, dass die Klemmleiste zum Vorlegen des einzulegenden Fadens (F) an den Einlegearm entsprechend verschoben, und dass die Düse (13) aktiviert wird.

12. Mechanismus nach Anspruch 11, dadurch gekennzeichnet, dass nach dem Vorlegen des Fadens (F) eine Verschwenkung des Einlegearms (14) aus der Arbeits- in die Ruhestellung und nach Erreichen der letzteren die Freigabe des Fadens durch die Klemme (3) erfolgt.

## Claims

1. A mechanism for inserting a thread into a yarn tester which has a test stage and a take-over member (13) for the inserted thread (F), with an insertion arm (14) and with a clamp (3) for feeding the thread (F) with the latter, characterised in that the insertion arm (14) is designed as a pivoting arm mounted at a fixed location, and the thread (F) is fixed in the clamp (3) until transfer to the take-over member (13) and is supplied to the take-over member as a loop.

2. A mechanism according to Claim 1, characterised in that a thread guide (2) is arranged in front of the clamp (3) in the thread running direction, and the thread (F) is clamped between the thread guide (2) and the clamp and is fed to the insertion arm (14) in this region.

3. A mechanism according to Claim 2, characterised in that the clamp (3) and thread guide (2) are carried by a common carrier, and in that a multiplicity of such clamps, thread guides and carriers, which form a displaceable clamping strip (1), is provided.

4. A mechanism according to one of Claims 1 to 3, characterised in that the take-over member (13) is formed by an air nozzle, preferably a suction nozzle, and in that the thread (F) is guided into the region of the entry orifice of the nozzle by the insertion arm (14).

5. A mechanism according to Claim 4, characterised in that the insertion arm (14) is made angled, preferably L-shaped, and has two legs and at its one end is fastened to a driveable carrier (16) and at its other end is provided with a thread guide (19).

6. A mechanism according to Claim 5, characterised in that the leg of the insertion arm (14) connected to the carrier (16) is oriented parallel to the connecting axis between the clamp (3) and the nozzle (13) and the other leg is oriented at right angles thereto and in that the path of the insertion arm during its pivoting movement from the clamp to the nozzle amounts to approximately 180°.

7. A mechanism according to Claim 6, characterised in that switches (21, 22) actuable by the insertion arm (14) are provided for detecting when the respective end position of the pivoting movement is reached.

8. A mechanism according to Claims 3 and 7, characterised in that the carrier (16) of the insertion arm (14), the clamping strip (1) and the nozzle (13) are arranged on a common plate (11).

9. A mechanism according to Claim 8, characterised in that the plate (11) is provided with a recess (10) which has a groove (20) for receiving the insertion arm (14), and in that the recess is closed off at the top by a pivotable flap or door (12).

10. A mechanism according to Claim 9, characterised in that, after the insertion operation, the insertion arm (14) is positioned in its position of rest with the thread guide (19) in the region of the nozzle (13).

11. A mechanism according to Claim 10, characterised in that, when the mechanism is put into operation, a pivoting of the insertion arm (14) out of the position of rest into the working position, with the thread guide (19) in the region of the clamping strip (1), takes place, in that the clamping strip is correspondingly displaced for feeding the thread (F) to be inserted to the insertion arm, and in that the nozzle (13) is activated.

12. A mechanism according to Claim 11, characterised in that, after the feed of the thread (F), a pivoting of the insertion arm (14) out of the working position into the position of rest takes place, and after the latter is reached the release of the thread by the clamp (3) takes place.

## Revendications

1. Mécanisme pour insérer un fil dans un appareil de contrôle de fil qui présente un trajet de contrôle et un organe de réception (13) pour le fil inséré (F), avec un bras d'insertion (14) et une pince (3) pour la présentation du fil (F) à celui-ci, caractérisé en ce que le bras d'insertion (14) est réalisé comme bras pivotant logé d'une manière fixe et que le fil (F), jusqu'à la transmission à l'organe de réception (13), est fixé dans la pince (3) et est amené à l'organe de réception comme boucle.

2. Mécanisme selon la revendication 1, caractérisé en ce qu'il est disposé dans la direction du périple du fil devant la pince (3) un guide-fil (2) et que le fil (F) est tendu entre celui-ci et la pince et est présenté dans cette zone au bras d'insertion (14).

3. Mécanisme selon la revendication 2, caractérisé en ce que la pince (3) et le guide-fil (2) sont supportés par un support commun et en ce qu'il est prévu une pluralité de telles pinces, guide-fils et supports qui forment une baguette de serrage déplaçable (1).

4. Mécanisme selon l'une des revendications 1 à 3, caractérisé en ce que l'organe de réception (13) est formé par une buse d'air, de préférence une buse d'aspiration, et en ce que le fil (F) est guidé par le bras d'insertion (14) dans la zone de l'ouverture d'entrée de la buse.

5. Mécanisme selon la revendication 4, caractérisé en ce que le bras d'insertion (14) est réalisé d'une manière coudée, de préférence en forme de L et présente deux branches et est fixé à l'une de ses extrémités à un support (16) pouvant être entraîné et est pourvu à son autre extrémité d'un guide-fil (19).

6. Mécanisme selon la revendication 5, caractérisé en ce que la branche du bras d'insertion (14) reliée au support (16) est orientée parallèlement à l'axe de liaison entre la pince (3) et la buse (13) et l'autre branche perpendiculairement à celui-ci, et en ce que le trajet du bras d'insertion, lors de son mouvement de pivotement de la pince à la buse est environ de 180°.

7. Mécanisme selon la revendication 6, caractérisé en ce que sont prévus des commutateurs (21, 22) actionnables par le bras d'insertion (14) pour détecter l'atteinte de la position d'extrémité respective du mouvement de pivotement.

8. Mécanisme selon les revendications 3 et 7, caractérisé en ce que le support (16) du bras d'insertion (14), la baguette de serrage (1) et la buse (13) sont disposés sur une plaque commune (11).

9. Mécanisme selon la revendication 8, caractérisé en ce que la plaque (11) présente un creux (10) qui présente une rainure (20) pour recevoir le bras d'insertion (14), et en ce que le creux est fermé en haut par un volet ou une porte pivotante (12).

10. Mécanisme selon la revendication 9, caractérisé en ce que le bras d'insertion (14), après l'opération d'insertion, est positionné dans sa position de repos avec le guide-fil (19) au voisinage de la buse (13).

11. Mécanisme selon la revendication 10, caractérisé en ce que lors de la mise en fonctionnement du mécanisme, il est provoqué un pivotement du bras d'insertion (14) de la positon de repos dans la position de travail avec le guide-fil (19) au voisinage de la baguette de serrage, en ce que la baguette de serrage pour présenter le fil à insérer (F) au bras d'insertion, est déplacée de manière correspondante et que la buse (13) est activée.

12. Mécanisme selon la revendication 11, caractérisé en ce que, après la présentation du fil (F), a lieu un pivotement du bras d'insertion (14) de la position de travail dans la position de repos et après l'atteinte de cette dernière, la libération du fil par la pince (3).
